# EUROPEAN PATENT APPLICATION

(11) **EP 3 895 692 A1**
(43) Date of publication of application: **20.10.2021**
(21) Application number: 20169697.8
(22) Date of filing: 15.04.2020
(51) Int. Cl.: A61K 9/00, A61K 31/00, A61K 47/10

(54) **ORAL THIN FILM WITH SMOOTH FUSED FILM**

(71) Applicant: LTS Lohmann Therapie-Systeme AG, 56626 Andernach (DE)
(72) Inventor: Linn, Michael, 55596 Waldböckelheim (DE); Warnus, Sabine, 56729 Ettringen (DE); Norelli, Claudia, 56204 Hillscheid (DE); Ficker, Mario, 53179 Bonn (DE)
(74) Representative: Held, Stephan

(57) **Abstract**

The invention relates to an oral thin film, comprising a polymer matrix and at least one water-insoluble, particulate active pharmaceutical ingredient dispersed in the polymer matrix, wherein the matrix polymer is poly(ethylene oxide) having a melting point of at least 55 °C, the amount of poly(ethylene oxide) having a melting point of at least 55 °C is at least 40% by weight, based on the total weight of the oral thin film, and wherein the oral thin film is obtainable by a process comprising the steps of: a) preparing a suspension comprising the matrix polymer, the at least one water-insoluble, particulate active pharmaceutical ingredient and a solvent which is water or a mixture of water and one or more organic solvents, b) casting the suspension obtained on a support or in a mold, and c) drying the suspension at a temperature above the melting temperature of the poly(ethylene oxide) to obtain the oral thin film.

The oral thin film is suitable as a medicament and can be administered in the oral cavity without addition of drinking water. The oral thin film has a very smooth surface providing a good mouth feel. Moreover, an enhanced protection of the active pharmaceutical ingredient against external influences is achieved.

## Description

The invention relates to oral thin films including a water-insoluble, particulate active pharmaceutical ingredient suitable as a medicament for oral administration.

Oral thin films (OTF) are thin, flexible films based on a polymer matrix and loaded with active substances for drug delivery. The oral thin films are taken orally and dissolve immediately in the mouth or are applied to the mucosa. They are placed on or under the tongue, or buccal where they then dissolve or disintegrate.

A common dosage form of a drug is a tablet. For instance, ulipristal acetate is a well-known emergency contraceptive ("morning-after pill") which is administered as a tablet ("EllaOne®") containing 30 mg micronized ulipristal acetate and lactose monohydrate, povidone, croscarmellose sodium and magnesium stearate as further ingredients. EllaOne® was approved in the European Union in 2009. EP 422100 B1 discloses the active pharmaceutical ingredient ulipristal acetate.

Tablets like EllaOne® are usually taken with water to ease swallowing. In regions where there is no quick access to clean drinking water, taking tablets might thus be challenging, especially for certain patient groups having difficulties with swallowing medications i.e. suffering from dysphagia. Administration of oral thin films, which quickly dissolve upon application in the oral cavity, does not require additional water and is therefore advantageous.

WO 2008/089151 A2 relates to a film incorporating high amounts of pharmaceutical agents and a polymer and methods for the preparation of the same. The examples mentioned for the polymer inter alia include polyethylene oxide. The film may be formed by a controlled drying process in order to achieve uniformity of the film. Desirably, the films contain a pharmaceutical active agent with no more than a 10 % variance of the active agent per unit area of the film. In the examples, films with a grainy taste are obtained.

To achieve high patient compliance for administration of an oral thin film, a polymer matrix is necessary, which binds the active pharmaceutical ingredient (e.g. ulipristal acetate), creates a pleasant sensation in the oral cavity upon administration (pleasant "mouthfeel") and adheres well to the mucosa.

Since active pharmaceutical ingredients are often sensitive to ambient conditions, a kind of protection or stabilization is also desirable.

Accordingly, the object of the present invention was to provide an oral thin film, which stably encloses the active pharmaceutical ingredient and creates a good mouth feeling with no or only low foreign body sensation. In addition, the oral thin film should adhere well to the oral mucosa.

An oral thin film as defined in claim 1 can achieve this object. Accordingly, the present invention relates to an oral thin film, comprising a polymer matrix and at least one water-insoluble, particulate active pharmaceutical ingredient dispersed in the polymer matrix, wherein the matrix polymer is poly(ethylene oxide) having a melting point of at least 55 °C, the amount of poly(ethylene oxide) having a melting point of at least 55 °C (DSC melting point (peak) temperature) is at least 40% by weight, based on the total weight of the oral thin film, and wherein the oral thin film is obtainable by a process comprising the steps of:
a) preparing a suspension comprising the matrix polymer, the at least one water-insoluble, particulate active pharmaceutical ingredient and a solvent which is water or a mixture of water and one or more organic solvents,
b) casting or coating the suspension obtained on a support, coating liner or in a mold, and
c) drying the suspension at a temperature above the melting temperature of the poly(ethylene oxide) to obtain the oral thin film.

It has been found that drying of the suspension containing the poly(ethylene oxide) matrix polymer and the active pharmaceutical ingredient leads to short-term melting of the polymer during the drying period with the active pharmaceutical ingredient dispersed therein. As a result, the active pharmaceutical ingredient (e.g. ulipristal acetate) is closely embedded in the polymer matrix and protected against external influences.

Furthermore, such a process creates a surface of the oral thin film which is very smooth (similar to a smooth plastic foil) which provides a good mouth feel upon intake in the oral cavity. At the same time, the oral thin film adheres well to the mucosa due to the low glass transition temperature of poly(ethylene oxide).

API is a common abbreviation for an active pharmaceutical ingredient. As indicated, oral thin films in general are known and abbreviated as OTF. Oral thin films that readily dissolve in the oral cavity are also commonly referred as orodispersible films.

The OTF of the invention have e.g. a size in the range of 0.3 to 20 cm², preferably 1 to 10 cm². The thickness of the OTF may be e.g. in the range of 10 to 1000 µm, preferably 40 to 400 µm. The OTF of the invention can take the form of a single-layer or multi-layer film, wherein a single-layer film is preferred.

The OTF of the invention comprises a polymer matrix and at least one water-insoluble, particulate active pharmaceutical ingredient dispersed in the polymer matrix.

Herein, a water-insoluble API refers to an API having a solubility in water of not more than 1.0 g/L, preferably not more than 0.3 g/L, at a temperature of 25 °C.

In general, the API is a lipophilic API. Herein, a lipophilic API refers to an API having a log P (n-octanol/water partition coefficient) of more than 1.5, preferably more than 2.5, at a temperature of 25 °C.

Suitable APIs are consequently, inter alia, agents for treating infection; virostatics; analgesics such as fentanyl, sufentanil, buprenorphine; anaesthetics; anorectics; active ingredients for the treatment of arthritis and asthma, such as terbutaline; anticonvulsants; antidepressants; antidiabetics; antihistamines; antidiarrhoeics; agents against migraines, itching, sickness and nausea; travel sickness, such as scopolamine and ondansetron; Parkinson's drugs; antipsychotics; antipyretics, spasmolytics, anticholinergics, agents against ulcers, such as ranitidine; sympathomimetics; calcium channel blockers such as nifedipine; betablockers; beta agonists such as dobutamine; antiarrhythmics; antihypertonics; ACE inhibitors; benzodiazepine agonists such as flumazenil; coronary, peripheral and cerebral vasodilators; stimulation for the central nervous system; hormones; hypnotics; immunosuppressants; muscle relaxants; N-methyl D aspartate (NMDA) receptor antagonists; parasympatholytics; parasympathomimetics; prostaglandins; psychostimulants; sedatives; tranquilizers; cough suppressant such as dextromethorphan.

In a preferred embodiment, the at least one water-insoluble, particulate API is selected from hormones, terpenes, hormone analogues, opioids, nonsteroidal anti-inflammatory drugs (NSAIDS), dopamine receptor agonist, antipsychotics, anticholinergic, synthetic opioids and/or imidazolines, preferably hormones. Examples of suitable hormones are steroid hormones or prostaglandins. Examples for NSAIDS are ibuprofen and ketoprofen.

In a preferred embodiment, the at least one water-insoluble, particulate active pharmaceutical ingredient is selected from ulipristal acetate, ibuprofen or ketoprofen. In a particular preferred embodiment, the at least one water-insoluble, particulate active pharmaceutical ingredient is ulipristal acetate. The ulipristal acetate is preferably micronized ulipristal acetate.

Ulipristal acetate is 17α-acetoxy-11α-(4-N,N-dimethylaminophenyl)-19-norpregna-4,9-dien-3,20-dion) with the following chemical formula:

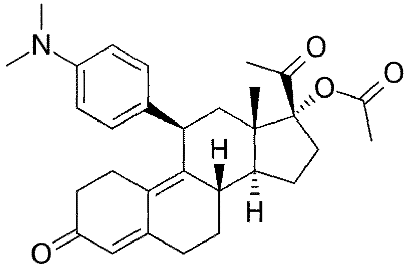

The particulate, water-insoluble API is dispersed in the polymer matrix. The API is preferably in crystalline form. The particles are firmly attached to the polymer matrix. According to an optical evaluation, an API such as ulipristal acetate is embedded in the matrix in such a way that a homogeneous OTF is obtained (no "API crumbs").

The amount of the at least one water-insoluble particulate API in the oral thin film is preferably 8 to 60% by weight, more preferably 15 to 40% by weight, based on the total weight of the oral thin film.

The polymer of the matrix of the oral thin film is poly(ethylene oxide) having a melting point of at least 55 °C Poly(ethylene oxides) are water-soluble polymers. One, two or more types of poly(ethylene oxide) may be used, but preferably one type of poly(ethylene oxide) is used. In general, a suitable poly(ethylene oxide) has a melting point in the range of 55 to 75 °C.

The melting point as used herein is defined as the melt peak temperature as measured by differential scanning calorimetry (DSC). DSC was conducted on a DSC 204 F1 Phoenix® (Netzsch) applying a starting temperature of -60 °C to an end temperature of +210 °C with a heating rate of 10 K/min (2 heating cycles) under nitrogen atmosphere (20 ml/min nitrogen) in an aluminum crucible.

The poly(ethylene oxide) used as water-soluble polymer preferably has a molecular weight in the range of 50,000 to 180,000 Dalton, preferably 75,000 to 150,000 Dalton. A particular preferred poly(ethylene oxide) is poly(ethylene oxide) WSR N-10 (PEO WSR N-10) having a molecular weight of about 100,000 Dalton and a melting point of about 65 °C. PEO WSR N-10 is commercially available as Polyox® WSR N-10 or Polyox® WSR N-10 NF, respectively from Dow Chemical Company.

The amount of poly(ethylene oxide) having a melting point of at least 55 °C, preferably PEO WSR N-10, is at least 40% by weight, based on the total weight of the oral thin film, wherein the amount is preferably 40 to 85% by weight or 50 to 85% by weight, more preferably 55 to 82% by weight, based on the total weight of the oral thin film.

In a preferred embodiment, the weight ratio of matrix polymer to the at least one water-insoluble, particulate active pharmaceutical ingredient (PEO/API) is in the range of 1/1 to 10/1, preferably 1.6/1 to 2.8/1, more preferably 1.8/1 to 2.4/1.

The high proportion of poly(ethylene oxide) matrix and the high PEO/API ratio, respectively, in the oral thin film according to the invention, together with a drying step at a temperature above the melting point of the polymer matrix enables the API being closely embedded in the polymer matrix so that an improved protection of the API against external influences is achieved. This is advantageous in view of storage stability and inhibition of degradation reactions, respectively.

In a particular preferred embodiment, the oral thin layer further comprises one or more plasticizers. Examples for suitable plasticizers are polyols, such as glycerol, diethylene glycol, polyethylene glycol, propylene glycol, dipropylene glycol or glycerol monoesters with fatty acids, and glycerol triesters such as triacetin, esters of citric acid such as triethyl citrate, acetyltributylcitrate, water, ethanol, α-tocopherol benzyl benzoate, butyl stearate, chlorobutanol, dibutyl phthalate, dimethyl phthalate, diethyl phthalate, dibutyl sebacate, stearic acid, tricaprylin. The plasticizer is preferably glycerol and/or triacetin.

The plasticizer mainly contributes to lowering the melting point and reducing the glass transition temperature. The addition of the plasticizer enhances the mucoadhesive properties of the oral thin film. A further benefit is that haptically flexible OTF can be obtained by addition of the plasticizer.

If present, the total amount of plasticizer, preferably glycerol and/or triacetin, is usually in the range of 0.5 and 20% by weight, preferably 3 to 7% by weight, based on the total weight of the oral thin film.

The oral thin layer may further comprise one or more further excipients, which are common in this technical field. Examples for suitable excipients are taste-masking agents, sweetening agents, flavoring agents, lubricants, pigments, coloring agents, stabilizers, fillers, saliva stimulating agents, emulsifiers, surfactants, enhancers, pH regulating agents, buffers, buffering agents, release modifiers, softeners, moisturizers, mold release agents, adhesives, anti-adherents and antioxidants. Preferably, one or more sweetening agents are used in the oral thin film. Examples for sweetening agents are sodium saccharin and sucralose. The total amount of optional further excipients, such as sweetening agents, is usually not more 15% by weight, preferably not more than 5% by weight, based on the total weight of the oral thin film.

Examples for antioxidants are sodium metabisulfite, butylated hydroxytoluene (BHT), butylated hydroxyanisole (BHA), ascorbic acid, tocopherols.

The residual solvent content in the oral thin film obtained, i.e. after drying step c), is preferably in the range of 0.2 to 10% by weight, preferably 0.8 to 6% by weight, based on the total weight of the oral thin film. The residual solvent contained may be water or water and one or more organic solvents.

The oral thin film is obtainable by a process comprising the steps of:
a) preparing a suspension comprising the matrix polymer, the at least one water-insoluble, particulate active pharmaceutical ingredient and a solvent which is water or a mixture of water and one or more organic solvents,
b) casting or coating the suspension obtained on a support, coating liner or in a mold, and
c) drying the suspension at a temperature above the melting temperature of the poly(ethylene oxide) to obtain the oral thin film.

The materials of the oral thin film to be prepared and suitable proportions thereof have been discussed above to which reference is made.

At first, a suspension comprising the matrix polymer, the at least one water-insoluble, particulate API and a solvent which is water or a mixture of water and one or more organic solvents, is prepared. In the suspension, the at least one water-insoluble, particulate API is suspended in the solvent. The matrix polymer is generally dissolved in the solvent.

The solvent used is water or a mixture of water and one or more organic solvents, wherein use of water is preferred. Examples for a suitable organic solvent are organic solvents that are miscible with water, such as alcohols, in particular ethanol and ethylene glycol, or ketones, in particular acetone, or ethers, in particular tetrahydrofuran and 1,4-dioxane. In case a mixture of water and one or more organic solvents is used, the weight ratio of water to organic solvent, preferably ethanol, may be e.g. in the range of 95/5 to 30/70.

In a preferred embodiment, one or more plasticizers, preferably glycerol and/or triacetin, are incorporated in the suspension. Optionally, one or more further excipients such as sweetening agents may be added at any order.

Any order for combining the ingredients to prepare the suspension is suitable. For instance, the matrix polymer may be dissolved in the solvent, and then the at least one water-insoluble, particulate API may be added to prepare the suspension. One or more plasticizers and/or one or more further excipients may be added before, during or after addition of the at least one API.

The OTF of the invention may be a non-foamed or non-porous film. Alternatively, the OTF can comprise a matrix present in the form of a solidified foam having spaces or cavities that are filled with a gas, a gas mixture, a liquid or a liquid mixture. Such OTFs are commonly referred to as "foam-OTFs".

In case of preparing a foam OTF (foamed film), the suspension is generally foamed with a gas. Foaming is generally carried out before casting step b). Examples for a suitable gas for foaming are air, argon, N₂, or CO₂.

The suspension obtained is casted or coated on a support, coating liner or in a mold. The suspension may spread by itself and/or the suspension is spread to the desired wet thickness. Preparation of the suspension and casting or coating and optional spreading are common process steps known by the skilled person.

The suspension casted on the support or in the mold and optionally spread is dried at a temperature above the melting temperature of the poly(ethylene oxide). In the drying step, the solvent is evaporated to obtain the oral thin film. The films obtained can be cut into pieces of desired dimensions.

In a preferred embodiment, the suspension is dried at a temperature which is at least 2 °C higher, preferably at least 4°C higher than the melting temperature of the poly(ethylene oxide). Alternatively or in addition, it is preferred that the suspension is dried at a temperature which is not more than 30 °C higher, preferably not more than 20 °C higher than the melting temperature of the poly(ethylene oxide). The suspension is preferably dried at a temperature in the range of 60 to 80 °C, preferably 65 to 75 °C.

The thermal treatment for drying the suspension may be carried out e.g. for 4 to 60 min, preferably 8 to 30 min.

For instance, in the preferred embodiment where PEO WSR N10 (Polyox®WSR N10) having a melting point of approximately 65 °C is used, drying the suspension at a temperature of about 70 °C is suitable.

In the present invention poly(ethylene oxides) were used whose melting point was below the drying temperature of the suspension. This ensured a brief fusing of the film matrix during drying. As a result of this, a very smooth film is produced which stably encloses the API, e.g. ulipristal acetate. The produced oral thin film with a very smooth surface (similar to a smooth plastic foil) provides a good mouth feel upon oral administration. As the API is suspended during melt of the polymer matrix, a close encasement of the API in the polymer matrix is achieved which after solidification yields an enhanced protection for the API against external influences.

Accordingly, it is preferred that the surface roughness Ra on both sides of the oral thin film is less than 1 µm, preferably less than 0.8 µm, more preferably less than 0.4 µm, and/or that the surface roughness Ra on at least one side of the oral thin film is less than 0.3 µm.

Moreover, the poly(ethylene oxides) used have glass transition temperatures below the body temperature (<37 °C) which ensures good adhesion in the oral cavity. The oral thin films provide a good adhesion to the mucosa.

The invention also relates to method for preparing an oral thin film according to the invention which comprises a polymer matrix and at least one water-insoluble, particulate active pharmaceutical ingredient dispersed in the polymer matrix, wherein the matrix polymer is poly(ethylene oxide) having a melting point of at least 55 °C, the amount of poly(ethylene oxide) having a melting point of at least 55 °C is at least 40% by weight, based on the total weight of the oral thin film, and wherein the process comprises the steps of:
a) preparing a suspension comprising the matrix polymer, the at least one water-insoluble, particulate active pharmaceutical ingredient and a solvent which is water or a mixture of water and one or more organic solvents,
b) casting or coating the suspension obtained on a support, coating liner or in a mold, and
c) drying the suspension at a temperature above the melting temperature of the poly(ethylene oxide) to obtain the oral thin film.

Details on materials used, suitable proportions thereof and the process steps have been discussed above so that reference is made thereto.

The invention also relates to an oral thin film according to the invention as described above for use as a medicament. If the API includes ulipristal acetate, the oral thin film is suitable for use as an emergency contraceptive, i.e. for use in the prevention of pregnancy after sex, in particular after unprotected sex.

The oral film will be administered in the oral cavity where fast disintegration of the film with release of the API is achieved. Addition of drinking water is not necessary.

The invention will now be explained more specifically with reference to the following examples, which are given for illustration of this invention and are not intended to be limiting thereof.

### Examples

In all examples with ulipristal acetate as API, ulipristal acetate having the following particle size distribution was used:
d₁₀ = 1.48 µm ± 11.20%; d₅₀ = 3.64 µm ± 8.71%; d₉₀ = 6.73 µm ± 10.40%; d₉₅ = 7.86 µm ± 11.08%; d₉₉ = 10.52 µm ± 16.30%.

### Example 1 - PEO-based OTF with ulipristal acetate as API

OTF laminates were prepared by standard laboratory methods (stirrers, glass vessels, coating tools, drying oven). The formulations were prepared as suspension formulations by mixing API, matrix polymer and excipients in a process solvent (water) for a suitable time, then coating the prepared mass on a suitable liner, followed by drying in a drying oven. This process yielded laminate pieces that were punched into OTF of a suitable size. Polyox WSR N10 was used as pre-solution (Polyox WSR N10: 21 % in water).

An oral thin film having the following formulation (stated as dry basis) was prepared as suspension formulation with water as process solvent (solid content 30 %), and a drying temperature of 70 °C:

| **ingredient** | **function** | **proportion [% by weight]** |
|---|---|---|
| Polyox® WSR N-10 | matrix polymer | 62.0 |
| ulipristal acetate (micronized) | API | 30.0 |
| glycerol | plasticizer (reduces melting temperature and glass transition temperature) | 5.0 |
| sodium saccharin | sweetening agent | 2.0 |
| sucralose | sweetening agent | 1.0 |

A tear resistant oral thin film was achieved. A haptical and optical assessment of the OTF obtained reveals a very smooth film surface and good embedding of the API in the polymer matrix.

### Example 2 - PEO-based OTF with ulipristal acetate as API, alternative process solvent mixture

OTF laminates were prepared by standard laboratory methods (stirrers, glass vessels, coating tools, drying oven). The formulations were prepared as suspension formulations by mixing API, matrix polymer and excipients in a process solvent (ethanol/water 10%/90% mixture) for a suitable time, then coating the prepared mass on a suitable liner, followed by drying in a drying oven. This process yielded laminate pieces that were punched into OTF of a suitable size.

An oral thin film having the following formulation (stated as dry composition) was prepared as suspension formulation with water as process solvent (solid content 30 %), and a drying temperature of 70 °C:

| **ingredient** | **function** | **proportion [% by weight]** |
|---|---|---|
| Polyox® WSR N-10 | matrix polymer | 62.0 |
| ulipristal acetate (micronized) | API | 30.0 |

| | | |
|---|---|---|
| glycerol | plasticizer (reduces melting temperature and glass transition temperature) | 5.0 |
| sodium saccharin | sweetening agent | 2.0 |
| sucralose | sweetening agent | 1.0 |

A tear resistant oral thin film was achieved. A haptical and optical assessment of the OTF obtained reveals a very smooth film surface and good embedding of the API in the polymer matrix.

The following table shows melting point (peak temperature) of OTF sample from example 1 (3 measurements). There is still residual moisture and/or residual plasticizer in the film. This can lower the melting point compared to the pure polymer (melting point lowering).

| | **Measurement:** | **1** | **2** | **3** |
|---|---|---|---|---|
| **Peak 1** | Peak Onset [°C] | 51.2 | 50.6 | 51.6 |
| | Peak temperature [°C] | 59.6 | 61.5 | 64.7 |
| | Peak End [°C] | 65.3 | 69.6 | 71.7 |
| **Peak 2** | Peak Onset [°C] | 169.6 | 169.6 | 169.5 |
| | Peak temperature [°C] | 177.1 | 177.2 | 177.2 |
| | Peak End [°C] | 181.2 | 181.8 | 181.8 |

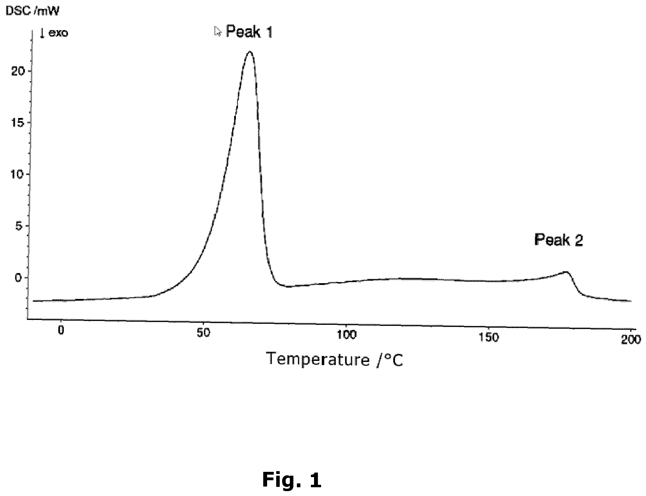

Figure 1 shows the DSC of Example 1 OTF showing the melting peak of the Polyox WSR N10 matrix polymer (Peak 1) and the melting peak of the API ulipristal acetate (Peak 2). As mentioned above melting point lowering may occur due to moisture/plasticizer residues.

### Example 3 - PEO-based OTF with ibuprofen as API

OTF laminates were prepared by standard laboratory methods (stirrers, glass vessels, coating tools, drying oven). The formulations were prepared as suspension formulations by mixing API, matrix polymer and excipients in a process solvent for a suitable time, then coating the prepared mass on a suitable liner, followed by drying in a drying oven. This process yielded laminate pieces that were punched into OTF of a suitable size. Polyox WSR N10 was used as pre-solution (Polyox WSR N10: 21 % in water).

An oral thin film having the following formulation (stated as dry composition) was prepared as suspension formulation with water as process solvent (solid content 24 %), and a drying temperature of 70 °C:

| **ingredient** | **function** | **proportion [% by weight]** |
|---|---|---|
| Polyox® WSR N-10 | matrix polymer | 82.0 |
| Ibuprofen | API | 10.0 |
| glycerol | plasticizer (reduces melting temperature and glass transition temperature) | 5.0 |
| sodium saccharin | sweetening agent | 2.0 |
| sucralose | sweetening agent | 1.0 |

A tear resistant oral thin film was achieved. A haptical and optical assessment of the OTF obtained reveals a very smooth film surface and good embedding of the API in the polymer matrix.

### Example 4 - PEO-based OTF with ketoprofen as API

OTF laminates were prepared by standard laboratory methods (stirrers, glass vessels, coating tools, drying oven). The formulations were prepared as suspension formulations by mixing API, matrix polymer and excipients in a process solvent for a suitable time, then coating the prepared mass on a suitable liner, followed by drying in a drying oven. This process yielded laminate pieces that were punched into OTF of a suitable size. Polyox WSR N10 was used as pre-solution (Polyox WSR N10: 21 % in water).

An oral thin film having the following formulation (stated as dry composition) was prepared as suspension formulation with water as process solvent (solid content 35 %), and a drying temperature of 70 °C:

| **ingredient** | **function** | **proportion [% by weight]** |
|---|---|---|
| Polyox® WSR N-10 | matrix polymer | 82.0 |
| Ketoprofen | API | 10.0 |
| glycerol | plasticizer (reduces melting temperature and glass transition temperature) | 5.0 |
| sodium saccharin | sweetening agent | 2.0 |
| sucralose | sweetening agent | 1.0 |

A tear resistant oral thin film was achieved. A haptical and optical assessment of the OTF obtained reveals a very smooth film surface and good encasement of the API in the polymer matrix.

### Example 5 - Surface morphology measurement

Surface morphology measurement was performed with a KLA Tencor P15 surface profiler with a stylus tip radius of 2 µm. On both sides of an OTF of example 1 a surface scan was performed on an area of 2 mm x 2 mm. On this area 3 different line scans were measured and the Ra (arithmetic mean deviation of assessed profile), Rq (root mean squared), Rp (maximum peak hight) and Rv (maximum valley depth) amplitude parameters for surface roughness determined, which are depicted in table A and table B below. The values show that the two sides are both very smooth, with Ra values comparable to very smooth materials, such as polished steel.

**OTF side A :**

| Measurement | Ra/µm | Rq/µm | Rp/µm | Rv/µm |
|---|---|---|---|---|
| 1 | 0.11 | 0.14 | 0.39 | 0.57 |
| 2 | 0.12 | 0.14 | 0.30 | 0.79 |
| 3 | 0.10 | 0.14 | 0.28 | 0.85 |
| Average value | 0.10 | 0.14 | 0.32 | 0.74 |

**OTF side B :**

| Measurement | Ra/µm | Rq/µm | Rp/µm | Rv/µm |
|---|---|---|---|---|
| 1 | 0.10 | 0.12 | 0.32 | 0.54 |
| 2 | 0.11 | 0.14 | 0.56 | 0.37 |
| 3 | 0.12 | 0.15 | 0.31 | 0.54 |
| Average value | 0.11 | 0.14 | 0.40 | 0.48 |

### Comparative Example 1 - PVA-based OTF

An oral thin film having the following formulation (stated as dry composition) was prepared as suspension formulation with water as process solvent (solid content 40 %), air was used for foaming, and a temperature of 70 °C was applied for drying. The polymer used is PVA 4-88 (water-soluble polymer, molecular weight about 31,000 Dalton, degree of hydrolysis about 86.7-88.7 mol %, melting point / decomposition above 180 °C). The PVA 4-88 was used a pre-solution (PVA 4-88: 35 % in water)

| **ingredient** | **function** | **proportion [% by weight]** |
|---|---|---|
| PVA 4-88 | matrix polymer | 63.0 |
| ulipristal acetate (micronized) | API | 30.0 |
| glycerol | plasticizer (reduces melting temperature and glass transition temperature) | 4.0 |
| sodium saccharin | sweetening agent | 2.0 |
| sucralose | sweetening agent | 1.0 |

A haptical and optical assessment of the OTF obtained reveals a rough film surface.

**OTF side A :**

| Measurement | Ra/µm | Rq/µm | Rp/µm | Rv/µm |
|---|---|---|---|---|
| 1 | 1.14 | 1.52 | 4.45 | 6.65 |
| 2 | 1.32 | 1.68 | 5.36 | 4.62 |
| 3 | 1.69 | 2.15 | 4.08 | 12.87 |
| Average value | 1.38 | 1.78 | 4.63 | 8.05 |

**OTF side B :**

| Measurement | Ra/µm | Rq/µm | Rp/µm | Rv/µm |
|---|---|---|---|---|
| 1 | 2.17 | 2.84 | 13.24 | 6.18 |
| 2 | 2.45 | 3.18 | 9.94 | 7.26 |
| 3 | 2.50 | 3.05 | 8.50 | 7.03 |
| Average value | 2.37 | 3.02 | 10.56 | 6.82 |

### Comparative example 2 - Kollicoat®IR-based formulations

An oral thin film having the following formulation (stated as dry composition) was prepared as suspension formulation with water as process solvent (solid content 33.7 %), and a drying temperature of 70 °C. The polymer used is Kollicoat®IR (from BASF, a water-soluble polyvinyl alcohol/polyethylene glycol copolymer (melting point app. 208 °C).

| **ingredient** | **function** | **proportion [% by weight]** |
|---|---|---|
| Kollicoat®IR | matrix polymer | 66.0 |
| ulipristal acetate (micronized) | API | 30.0 |
| glycerol | plasticizer (reduces melting temperature and glass transition temperature) | 1.0 |
| sodium saccharin | sweetening agent | 2.0 |
| sucralose | sweetening agent | 1.0 |

A brittle oral thin film was achieved. A haptical and optical assessment of the OTF obtained reveals an uneven and non-continuous film surface and a poor embedding of the API in the polymer matrix.

### Comparative Example 3 PEO-based OTF with ulipristal acetate as API dried under melting point

OTF laminates were prepared by standard laboratory methods (stirrers, glass vessels, coating tools, drying oven). The formulations were prepared as suspension formulations by mixing API, matrix polymer and excipients in a process solvent (water) for a suitable time, then coating the prepared mass on a suitable liner, followed by drying in a drying oven. This process yielded laminate pieces that were punched into OTF of a suitable size. Polyox WSR N10 was used as pre-solution (Polyox WSR N10: 33 % in water).

An oral thin film having the following formulation (stated as dry basis) was prepared as suspension formulation with water as process solvent (solid content 30 %), and a drying temperature of 50 °C (below the melting point of Polyox WSR N10):

| **ingredient** | **function** | **proportion [% by weight]** |
|---|---|---|
| Polyox® WSR N-10 | matrix polymer | 62.0 |
| ulipristal acetate (micronized) | API | 30.0 |
| glycerol | plasticizer (reduces melting temperature and glass transition temperature) | 5.0 |
| sodium saccharin | sweetening agent | 2.0 |
| sucralose | sweetening agent | 1.0 |

A tear resistant oral thin film was achieved. A haptical and optical assessment of the OTF obtained reveals a mat film surface with agglomerated API.

A haptical and optical assessment of the OTF obtained reveals a smooth surface one side (side B) and rough and mat film surface on the other side (side A).

**OTF side A :**

| Measurement | Ra/µm | Rq/µm | Rp/µm | Rv/µm |
|---|---|---|---|---|
| 1 | 0.87 | 1.04 | 2.52 | 2.86 |
| 2 | 1.52 | 1.99 | 6.61 | 5.66 |
| 3 | 1.00 | 1.33 | 3.68 | 3.90 |
| Average value | 1.13 | 1.45 | 4.27 | 4.14 |

**OTF side B :**

| Measurement | Ra/µm | Rq/µm | Rp/µm | Rv/µm |
|---|---|---|---|---|
| 1 | 0.15 | 0.21 | 1.16 | 0.67 |
| 2 | 0.11 | 0.14 | 0.43 | 0.46 |
| 3 | 0.15 | 0.19 | 0.64 | 0.51 |
| Average value | 0.14 | 0.18 | 0.74 | 0.55 |

### Comparative Example 4 HPMC-based OTF with ulipristal acetate as API dried under melting point

OTF laminates were prepared by standard laboratory methods (stirrers, glass vessels, coating tools, drying oven). The formulations were prepared as suspension formulations by mixing API, matrix polymer (hydroxypropyl methylcellulose (HPMC) 603 and hydroxypropyl methylcellulose (HPMC) 60SH50) and excipients in a process solvent (water) for a suitable time, then coating the prepared mass on a suitable liner, followed by drying in a drying oven. This process yielded laminate pieces that were punched into OTF of a suitable size.

An oral thin film having the following formulation (stated as dry basis) was prepared as suspension formulation with water as process solvent (solid content 32 %), and a drying temperature of 70 °C (below the melting point of Polyox WSR N10):

| **ingredient** | **function** | **proportion [% by weight]** |
|---|---|---|
| hydroxypropyl methylcellulose (HPMC) 603 | matrix polymer | 30.40 |
| hydroxypropyl methylcellulose (HPMC) 60SH50 | matrix polymer | 16.37 |
| ulipristal acetate (micronized) | API | 39.80 |
| glycerol | plasticizer (reduces melting temperature and glass transition temperature) | 9.95 |
| sodium saccharin | sweetening agent | 0.64 |
| sucralose | sweetening agent | 0.32 |

A tear resistant oral thin film was achieved. A haptical and optical assessment of the OTF obtained reveals a mat film surface with agglomerated API.

A haptical and optical assessment of the OTF obtained reveals a mat surface one side (side A) and rough film surface on the other side (side B).

**OTF side A :**

| Measurement | Ra/µm | Rq/µm | Rp/µm | Rv/µm |
|---|---|---|---|---|
| 1 | 0.43 | 0.62 | 3.00 | 1.47 |
| 2 | 0.55 | 0.74 | 3.96 | 1.46 |
| 3 | 0.36 | 0.45 | 1.61 | 1.20 |
| Average value | 0.45 | 0.61 | 2.86 | 1.38 |

**OTF side B :**

| Measurement | Ra/µm | Rq/µm | Rp/µm | Rv/µm |
|---|---|---|---|---|
| 1 | 1.36 | 1.79 | 6.73 | 3.46 |
| 2 | 1.20 | 1.69 | 8.03 | 2.64 |
| 3 | 1.30 | 1.74 | 8.02 | 2.85 |
| Average value | 1.29 | 1.74 | 7.59 | 2.98 |

### Example 2 - Stability test

A stability test on the OTFs of example 1 and comparative example 1 was carried out by storing samples of the OTFs at a temperature of 40 °C and 75% relative humidity. After storage, the samples were tested for degradation products of ulipristal acetate by HPLC. The main degradation product detected was N-demethyl ulipristal acetate (DMUA). The following tables 1 and 2 show the amount of DMUA and the total amount of degradation product detected (Sum) in % by weight based on the initial amount of ulipristal acetate in the OTF before storage.

N-Demethyl ulipristal acetate (DMUA) has the following formula:

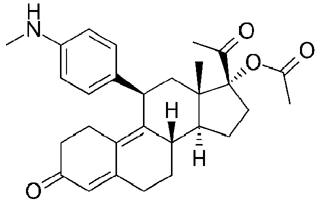

The formulation of example 1 was found more stable than the formulation of comparative Example 1. This shows a benefitting impact of the PEO as matrix polymer, where the API is closely embedded due to the manufacturing process.

Table 1: Summarized values form stability testing protocol for example 1 (PEO formulation) at 40 °C / 75% r.h.

| **Test Parameter** | **Inital** | **1 Month** | **2 Months** | **3 Months** | **6 Months** |
|---|---|---|---|---|---|
| Related substances | **Sum: 0.22** | **Sum: 0.26** | **Sum: 0.36** | **Sum: 0.43** | **Sum: 0.47** |
| N-Demethyl-Ulipristalacetat | 0.16 | 0.19 | 0.20 | 0.22 | 0.23 |
| Deprotector* | 0.02 | 0.02 | 0.02 | 0.02 | 0.03 |

| | | | | | |
|---|---|---|---|---|---|
| *Acetyl group removed ("deprotected") | | | | | |

Table 2: Summarized values form stability testing protocol for comparative example 1 (PVA formulation) at 40 °C / 75% r.h.

| **Test Parameter** | **Inital** | **1 Month** | **2 Months** | **3 Months** | **6 Months** |
|---|---|---|---|---|---|
| Related substances | **Sum: 0.21** | **Sum: 0.34** | **Sum: 0.64** | **Sum: 0.90** | **Sum: 1.17** |
| N-Demethyl-Ulipristalacetat | 0.16 | 0.23 | 0.32 | 0.37 | 0.48 |
| Deprotector* | 0.02 | 0.02 | 0.02 | 0.01 | 0.02 |

| | | | | | |
|---|---|---|---|---|---|
| *Acetyl group removed ("deprotected") | | | | | |

## Claims

1. An oral thin film, comprising a polymer matrix and at least one water-insoluble, particulate active pharmaceutical ingredient dispersed in the polymer matrix, wherein the matrix polymer is poly(ethylene oxide) having a melting point of at least 55 °C, the amount of poly(ethylene oxide) having a melting point of at least 55 °C is at least 40% by weight, based on the total weight of the oral thin film, and wherein the oral thin film is obtainable by a process comprising the steps of:
a) preparing a suspension comprising the matrix polymer, the at least one water-insoluble, particulate active pharmaceutical ingredient and a solvent which is water or a mixture of water and one or more organic solvents,
b) casting or coating the suspension obtained on a support, a coating liner or in a mold, and
c) drying the suspension at a temperature above the melting temperature of the poly(ethylene oxide) to obtain the oral thin film.

2. The oral thin film according to claim 1, wherein the at least one water-insoluble, particulate active pharmaceutical ingredient is selected from hormones, opioids, nonsteroidal anti-inflammatory drugs, dopamine receptor agonist, antipsychotics, anticholinergic, synthetic opioids terpenes, hormone analogues and/or imidazolines.

3. The oral thin film according to any preceding claim, wherein the at least one water-insoluble, particulate active pharmaceutical ingredient is selected from ulipristal acetate, ibuprofen or ketoprofen.

4. The oral thin film according to any preceding claim, wherein the amount of the at least one water-insoluble, particulate active pharmaceutical ingredient is 8 to 60% by weight, preferably 15 to 40% by weight, based on the total weight of the oral thin film.

5. The oral thin film according to any preceding claim, wherein
the poly(ethylene oxide) having a melting point of at least 55 °C has a molecular weight in the range of 50,000 to 180,000 Dalton, preferably 75,000 to 150,000 Dalton.

6. The oral thin film according to any preceding claim, wherein the poly(ethylene oxide) having a melting point of at least 55 °C is poly(ethylene oxide) WSR N-10.

7. The oral thin film according to any preceding claim, wherein
the amount of poly(ethylene oxide) having a melting point of at least 55 °C is 40 to 85% by weight, preferably 55 to 82% by weight, based on the total weight of the oral thin film.

8. The oral thin film according to any preceding claim, wherein the weight ratio of matrix polymer to the at least one water-insoluble, particulate active pharmaceutical ingredient (PEO/API) is in the range of 1/1 to 10/1, preferably 1.6/1 to 2.8/1, more preferably 1.8/1 to 2.4/1.

9. The oral thin film according to any preceding claim, further comprising one or more plasticizers, preferably glycerol and/or triacetin.

10. The oral thin film according to claim 9, wherein the amount of the one or more plasticizers, preferably glycerol and/or triacetin, is 0.5 to 20% by weight, preferably 3 to 7% by weight, based on the total weight of the oral thin film.

11. The oral thin film according to any preceding claim, wherein the solvent is a mixture of water and an alcohol, preferably ethanol, wherein the weight ratio of water to organic solvent, preferably ethanol, is preferably in the range of 95/5 to 30/70.

12. The oral thin film according to any preceding claim, wherein the suspension is dried at a temperature which is at least 2 °C higher, preferably at least 4°C higher than the melting temperature of the poly(ethylene oxide), and/or
wherein the suspension is dried at a temperature which not more than 30 °C higher, preferably not more than 20 °C higher than the melting temperature of the poly(ethylene oxide), and/or
wherein the suspension is dried at a temperature in the range of 60 to 80 °C, preferably 65 to 75 °C.

13. The oral thin film according to any preceding claim, wherein the suspension is foamed with a gas before step b).

14. The oral thin film according to any preceding claim, wherein
the surface roughness Ra on both sides of the oral thin film is less than 1 µm, preferably less than 0.8 µm, more preferably less than 0.4 µm, and/or
the surface roughness Ra on at least one side of the oral thin film is less than 0.3 µm.

15. A method for preparing an oral thin film comprising a polymer matrix and at least one water-insoluble, particulate active pharmaceutical ingredient dispersed in the polymer matrix, wherein the matrix polymer is poly(ethylene oxide) having a melting point of at least 55 °C, the amount of poly(ethylene oxide) having a melting point of at least 55 °C is at least 40% by weight, based on the total weight of the oral thin film, and wherein the process comprises the steps of:
a) preparing a suspension comprising the matrix polymer, the at least one water-insoluble, particulate active pharmaceutical ingredient and a solvent which is water or a mixture of water and one or more organic solvents,
b) casting or coating the suspension obtained on a support, coating liner or in a mold, and
c) drying the suspension at a temperature above the melting temperature of the poly(ethylene oxide) to obtain the oral thin film.

16. The method of claim 15, where the oral thin film and/or the process steps are as defined in claims 1 to 14.

17. An oral thin film according to one of claims 1 to 14 for use as a medicament.
